# EUROPÄISCHE PATENTSCHRIFT

(11) **EP 2 762 878 B1**
(45) Veröffentlichungstag und Bekanntmachung des Hinweises auf die Patenterteilung: **11.07.2018**
(21) Anmeldenummer: 14000361.7
(22) Anmeldetag: 31.01.2014
(51) Int. Cl.: G01N 33/46, G01N 33/38, G01N 29/07, G01N 29/265, G01N 3/40, G01N 29/04

(54) **Verfahren und Vorrichtung zur gesteuerten Beschaffenheitsuntersuchung von säulenförmigen oder zylindrischen Abschnitten von Körpern**
Method and device for guided examination of the condition of column-shaped or cylindrical sections of bodies
Procédé et dispositif d'analyse contrôlée de la condition de sections des corps cylindriques ou en forme de colonnes

(30) Priorität: 01.02.2013 DE 102013001711
(43) Veröffentlichungstag der Anmeldung: 06.08.2014
(73) Patentinhaber: IML-Instrumenta Mechanik Labor GmbH, 69168 Wiesloch (DE)
(72) Erfinder: Hunger, Sebastian, 69168 Wiesloch (DE); Hunger, Fabian, 69181 Leimen (DE); Hunger, Erich, 76131 Karlsruhe (DE)
(74) Vertreter: Drobnik, Stefanie

(56) Entgegenhaltungen:
- EP-A1- 2 549 272
- WO-A2-01/65253
- WO-A2-2007/052239
- DE-A1-102009 013 069
- DE-U1-202009 006 690

## Beschreibung

Die Erfindung betrifft ein Verfahren und eine Vorrichtung zur Beschaffenheitsuntersuchung von säulenförmigen oder zylindrischen Objekten bzw. derartigen Körpern.

Verfahren und entsprechende Vorrichtungen zur Beschaffenheitsuntersuchung sind unter anderem zur Untersuchung von Bäumen bekannt, da diese beispielsweise in urbanen Bereichen regelmäßig aus Gründen der Verkehrssicherheit auf mögliche vorliegende Defekte wie Fäulnis oder ähnliches untersucht werden müssen.

Nichtinvasive Verfahren wie die visuelle Analyse von Krone und Stamm sowie invasive Verfahren mit einer Probenmaterialentnahme aus dem Stamm und minimalinvasive Verfahren, bei denen z. B. mit einer in den Stamm eindringenden Bohrnadel eine Bohrwiderstandsmessung oder Impulslaufzeitmessung durchgeführt wird, sind in der DE 10 2009 013 069 beschrieben, die ein Verfahren zur Untersuchung der Beschaffenheit von Objekten wie Baumstämmen, aber auch zur Untersuchung von säulenförmigen Körpern oder Körpern mit säulenförmigen Abschnitten, die nicht aus Holz, sondern aus anderen Werkstoffen beschaffen sind, sowie entsprechende Vorrichtungen offenbart, die ein möglichst schädigungsarmes Eindringen einer Nadel in ein zu untersuchendes Objekt wie einen Holzstamm erlauben.

Dabei muss der Untersuchende selbst erwägen und begutachten, auf welche Weise die Untersuchung ausgeführt werden soll, was oft zu unzureichenden Ergebnissen führt, da der Untersuchende nicht in jedem Fall über hinreichende Erfahrung verfügt, um die Untersuchung mit dem zur Verfügung stehenden Gerät sachgerecht auszuführen.

Dasselbe gilt auch für den Gegenstand der DE 20 2009 006 690 U1. Diese betrifft eine Vorrichtung zur Ermittlung der Standsicherheit eins Masts, die keinen Bohrwiderstand misst, sondern die Eigenfrequenz des Masts, um daraus Rückschlüsse auf den inneren Zustand zu ziehen.

Ausgehend von diesem Stand der Technik liegt der vorliegenden Erfindung die Aufgabe zugrunde, eine Vorrichtung zu schaffen, mit der solche Beschaffenheitsuntersuchungen an säulenförmigen oder zylindrischen Objekten systematisiert und damit unabhängig von dem mit der Untersuchung beauftragten Bediener gemacht werden können.

Diese Aufgabe wird durch eine Vorrichtung mit den Merkmalen des unabhängigen Anspruchs 1 gelöst.

Die weitere Aufgabe der Schaffung eines entsprechenden Beschaffenheitsuntersuchungsverfahrens wird durch das Verfahren mit den Merkmalen des unabhängigen Anspruchs 5 gelöst. Bevorzugte Ausführungsformen werden durch die Unteransprüche beschrieben. Die erfindungsgemäße Vorrichtung, um Beschaffenheitsuntersuchungen an säulenförmigen oder zylindrischen Objekten wie beispielsweise Holzmasten auszuführen, umfasst ein Bohrgerät mit einer Bohrnadel und ferner Signalerfassungsmittel, um die durch die Bohrnadel, die in das zu untersuchende Objekt eingeführt wird, erzeugbaren oder aufnehmbaren Signale zu erfassen, sowie mehrere Drucksensoren, die so ausgestaltet sind, dass sie an dem Objekt angeordnet werden können; vorzugsweise umfänglich in einer Ebene. Vorteilhaft umfasst nun die Vorrichtung ferner eine Standort-Bestimmungseinrichtung und eine getrennt von dem Bohrgerät handhabbare Recheneinheit. Diese Recheneinheit und die Signalerfassungsmittel sind jeweils mit einer Kommunikationsschnittstelle zum Aufbau einer Kommunikationsverbindung ausgestattet, um die durch die Bohrnadel erzeugten oder aufgenommenen Signale von den Signalerfassungsmitteln an die Recheneinheit zu übertragen. Ferner hat die Recheneinheit wenigstens zumindest eine Schnittstelle zur Eingabe von Kennzeichnungsdaten des zu untersuchenden Objekts. Wenigstens der Standort des Objekts, der durch die Standort-Bestimmungseinrichtung bestimmt werden kann, soll dabei als relevanteste unter den Kennzeichnungsdaten stets eingegeben werden. Weiter umfasst die Recheneinheit bzw. der Computer eine Prozessor- und Speichereinheit mit einem Datenverarbeitungsprogramm konfiguriert zur Verarbeitung der Kennzeichnungsdaten des zu untersuchenden Objekts und zum Vorgeben eines Untersuchungsmodus für das zu untersuchende Objekt an einen Benutzer und zum Auswerten der durch die Bohrnadel erzeugten oder aufgenommenen Signale. Der Untersuchungsmodus umfasst dabei die Vorgabe einer Art wie die Messungen durchzuführen sind. Damit wird erreicht, dass der Benutzer bzw. Bediener der Vorrichtung sachgerecht durch den Untersuchungsablauf geführt wird und damit reproduzierbare und in der Sache zielführende korrekte Messergebnisse erzeugen kann. Ferner ist vorteilhaft eine Ausgabeeinheit vorgesehen, um den Untersuchungsmodus für das zu untersuchende Objekt vorzugeben und die Auswertungsergebnisse an den Benutzer auszugeben.

Generell ist denkbar, dass die mobile Recheneinheit und die Erfassungsmittel über ein Schnittstellenkabel verbunden werden. Bevorzugt jedoch wird die Datenübertragungsverbindung eine drahtlose Verbindung sein, die etwa über ein lokales Funknetz, beispielsweise Bluetooth® oder Wi-Fi aufgebaut wird.

Mit dieser Vorrichtung lassen sich die Beschaffenheitsuntersuchungen derart ausführen, dass sie unabhängig von dem ausführenden Bediener immer nach dem vorgegebenen System ablaufen und so die Verlässlichkeit der Überprüfung gesteigert werden kann.

Vorteilhaft kann die Standort-Bestimmungseinrichtung, die bevorzugt einen GPS-Empfänger aufweist, ebenso wie weitere Komponenten in das Bohrgerät integriert bzw. an die Steuerungselektronik des Bohrgeräts angebunden sein. Bei den weiteren Komponenten, die zur Durchführung des erfindungsgemäßen Verfahrens eingesetzt werden können, kann es sich um einen Kompass, wenn etwa an einem Objekt eine Bohrstelle in Bezug auf die Himmelsrichtung vorgegeben ist, und/oder eine Anzeigevorrichtung für den Einführwinkel einer durchzuführenden Bohrwiderstands- und Impulslaufzeitmessung handeln. Außerdem können die Erfassungsmittel an die Steuerelektronik des Bohrgeräts angebunden sein, die wiederum die Kommunikationsschnittstelle mit der mobilen Recheneinheit bereitstellen kann.

Eine alternative oder zusätzliche Ausführungsform kann vorsehen, dass die Standort-Bestimmungseinrichtung direkt in der Recheneinheit, die im Grunde eine mobile Recheneinheit ist und ein Notebook, Smartphone, Tablet-PC, Netbooks oder i-Pad sein kann, umfasst ist.

Darüber hinaus kann die Vorrichtung auch eine Bildaufnahmevorrichtung wie eine Kamera haben, die entweder in der Recheneinheit enthalten ist, oder zumindest mit dieser verbunden werden kann, etwa mittels eines Schnittstellenkabels oder auch drahtlos über eine Funkschnittstelle.

Das erfindungsgemäße Verfahren, das es ermöglicht, die Beschaffenheitsuntersuchung an den säulenförmigen oder zylindrischen Objekten des Interesses unter Verwendung einer erfindungsgemäßen Vorrichtung auszuführen, umfasst die Schritte
A) Ermitteln von Kennzeichnungsdaten, zumindest eines Standorts des Objekts, mit der Standortbestimmungseinheit und Erfassen der Daten durch die Recheneinheit,
B) einem Benutzer mit der Ausgabeeinheit Ausgeben eines Untersuchungsmodus für das zu untersuchende Objekt, dabei Vorgeben zumindest einer Bohrwiderstands- und Impulslaufzeitmessweise, einer Messhöhe über der Erdgleiche bzw. über dem Grund, und einen Einführwinkel und einer Mindestanzahl der an dem zu untersuchenden Objekt durchzuführender Messungen, und
C) Durchführen der Untersuchung gemäß dem vorgegebenen Untersuchungsmodus und Zuführen der aufgenommenen Messdaten, die zumindest Bohrwiderstands- und Impulslaufzeitmessdaten sind, dem Datenverarbeitungsprogramm der Recheneinheit und Auswerten derselben und
D) dem Benutzer Ausgeben des Auswertungsergebnisses.

Damit werden die Ergebnisse der quasi kombinierten Bohrwiderstands- und Impulslaufzeitmessung zuverlässig und reproduzierbar erzeugt, da dem Bediener Schritt für Schritt übermittelt wird, was zu tun ist und auf welche Weise.

In Schritt E) kann in Fortführung des Verfahrens in Abhängigkeit des Auswertungsergebnisses durch die Recheneinheit ausgewählt werden, ob die Untersuchung an dem Objekt fortzuführen ist und, falls ja, kann der weitere geeignete Untersuchungsmodus vorgegeben werden, nach dem dann wieder Schritte C) und D) erfolgen.

Die Standortangaben können im Übrigen absolute Standortangaben sein, wie geografische Koordinaten, die z. B. mittels GPS erhalten werden; oder es können relative Ortsangaben verwendet werden, die auf eine Adresse, auf Verkehrswege mit Kilometrierung, Richtungsangaben oder auf Geländedetails etc. bezogen sind. Es können auch beide, die absolute und die relative Ortsangabe des Objekts erfasst werden. Weitere Kennzeichnungsdaten können bei einem Mast z. B. eine Mastennummer sein, mit denen etwa Strommasten beschriftet sind. Damit können große Anzahlen nacheinander folgender Strommasten zielgerichtet und zuverlässig untersucht werden, auch wenn Messpausen zwischen der Untersuchung vorliegen.

Untersuchungsmodus meint dabei hierin die Art, wie die Messungen durchzuführen sind, etwa wie hoch über dem Grund bzw. der Erdgleiche, und der Einführwinkel, unter dem die Bohrnadel in das Objekt einzuführen ist.

Entsprechend diesen Vorgaben werden dann die kombinierten Bohrwiderstands- und Impulslaufzeitmessungen durchgeführt, wobei die aufgenommenen Impulslaufzeiten und Bohrwiderstandswerte einer Datenverarbeitungssoftware der mobilen Recheneinheit zugeführt werden, um vorteilhaft schnell und präzise die Auswertung vorzunehmen.

So kann das erfindungsgemäße Verfahren das Auswerten der gemäß der vorgegebenen Art und Mindestanzahl durchgeführten kombinierten Bohrwiderstands- und Impulslaufzeitmessungen mittels der Datenverarbeitungssoftware durch die Recheneinheit umfassen. Ferner kann in Abhängigkeit des Auswertungsergebnisses durch die mobile Recheneinheit ermittelt werden, ob weitere kombinierte Bohrwiderstands- und Impulslaufzeitmessungen an dem Objekt durchzuführen sind. Falls die Recheneinheit dies feststellt, insbesondere auch unter Berücksichtigung von entsprechenden Vergleichsdaten oder Vergleichsmesswerten, die auf dem Speicher abgelegt sind, so kann auch hierbei durch die Recheneinheit der weitere Untersuchungsmodus vorgegeben werden, dem folgend die weitere Untersuchung auszuführen ist.

Das Auswertungsergebnis, das die Beurteilung der Beschaffenheit des untersuchten Objekts umfasst, kann in der mobilen Recheneinheit abgelegt werden.

Alternativ oder zusätzlich kann das Auswertungsergebnis an dem untersuchten Objekt dokumentiert werden, indem etwa das untersuchte Objekt gekennzeichnet wird. So kann ein defektes Objekt wie etwa ein von Fäulnis befallener Holzmast mit einem Zeichen markiert werden, das diesen Beschaffenheitszustand des Holzmastes verdeutlicht, so dass der defekte Holzmast zum Austauschen schnell aufgefunden werden kann.

In einer weiteren Ausführungsform des Verfahrens kann nach der Erfassung der Kennzeichnungsdaten durch die Recheneinheit die Anweisung vorgegeben werden, eine visuelle Kontrolle und eine Dokumentation des zu untersuchenden Objekts durchzuführen, wobei die Dokumentation mit der Kamera erfolgen kann. Die Bilddaten können in die Auswertung einfließen, so dass die Recheneinheit bei der weiteren Empfehlung, ob die Untersuchung an dem Objekt fortzuführen ist, diese berücksichtigt. Die Kamera kann Teil der Recheneinheit sein. Alternativ kann vorgesehen sein, dass die Kamera mit der Recheneinheit verbunden wird.

Zur Durchführung der kombinierten Bohrwiderstands- und Impulslaufzeitmessung ist an sich vorgesehen, dass zunächst mehrere Sensoren zur Aufnahme von Drucksignalen in einer oder mehreren Ebenen umfänglich um das Objekt angeordnet werden. Das Messverfahren basiert auf dem diskontinuierlichen Einführen einer Bohrnadel mit Hilfe eines Bohrgeräts radial in Richtung der Längsachse des Objektabschnitts, der untersucht werden soll, wobei die Bohrnadel das quasi kombinierte Verfahren durchführt, indem sie, während sie eine Einführbewegung ausführt, den Bohrwiderstand im umgebenden Material nach bekannten Bestimmungsverfahren misst, sodann jedoch nach Zurücklegen eines Streckenabschnitts entlang der zurückzulegenden Strecke inne hält und erneut zur Fortführung der Bohrbewegung ansetzt, so dass durch die Unterbrechung ein Impuls induziert wird.

Dieser Impuls erzeugt im Inneren des Materials Impulswellen, die sich von der Quelle ausgehend ausbreiten. Abhängig von der Homogenität des Materials erfolgt die Ausbreitung mit einer definierten Ausbreitungsgeschwindigkeit, die im Wesentlichen auf Dichte und Härte des Materials zurückzuführen ist. Die Bohrnadel vollführt damit die Aufgabe eines Impulshammers, während sie gleichzeitig als Vorrichtung zur Bestimmung des Bohrwiderstands genutzt wird. Damit wird vorteilhaft während der Ausführung nur einer einzigen Tätigkeit die Erstellung zweier zur Materialbestimmung relevanter Datensätze möglich, nämlich der Impulslaufzeitmessung zur Erstellung eines Impulstomogramms und der Erstellung eines Bohrwiderstanddiagramms. Insofern, als die Impulswellen unmittelbar im Inneren, bis hin zur maximalen Eindringtiefe der Bohrnadel, erzeugt werden, ist es möglich, eine Abbildung der Beschaffenheit des Kernmaterials von hoher Präzision zu schaffen. Ferner werden in einer weiteren Ausführungsform die pro Streckenabschnitt gemessenen Widerstandswerte der Bohrwiderstandsmessung mit dem erhaltenen Impulstomogramm korreliert, wobei durch die Kombination der erhaltenen Messdaten, die unterschiedlicher Art sind, zugleich eine Aussage über Form und Größe und Lokalisierung eines möglichen Defekts sowie über die Art des Defekts ermittelt werden kann.

Das Verfahren kann an Objekten wie Bäumen und Holzmasten ebenso ausgeführt werden, wie an Objekten, die aus Stein oder anderen Materialien beschaffen sind.

Weitere Ausführungsformen, sowie einige der Vorteile, die mit diesen und weiteren Ausführungsformen verbunden sind, werden durch die nachfolgende ausführliche Beschreibung deutlich und besser verständlich. Unterstützend hierbei ist auch der Bezug auf die Figuren in der Beschreibung. Gegenstände oder Teile derselben, die im Wesentlichen gleich oder sehr ähnlich sind, können mit denselben Bezugszeichen versehen sein.
**Fig. 1a** zeigt eine Seitenansicht eines Baums bei der Verwendung eines Bohrgeräts mit Bohrnadel bei der Ausführung des aus DE 10 2009 013 069 bekannten kombinierten Bohrwiderstands- und Impulslaufzeitmessverfahrens.
**Fig. 1b** zeigt eine Seitenansicht eines Baums bei der Verwendung des Bohrgeräts aus Fig. 1a bei einer in Richtung der Wurzel des Baumes weisenden Probebohrung.
**Fig. 2a** zeigt eine Draufsicht auf das aus DE 10 2009 013 069 bekannte Bohrgerät mit daran angeordneten Aufzeichnungs- und Führungsmitteln.
**Fig. 2b** zeigt eine Seitenansicht der Vorrichtung aus Fig. 2a.
**Fig. 3a** zeigt beispielhaft einen möglichen Messkurvenverlauf bei Aufnahme von Druckimpulsen und Bohrwiderstandsmessung bei einer Untersuchung eines Baumstammes, wenn die Bohrnadel aus gesundem Holz auf Braunfäule trifft.
**Fig. 3b** zeigt beispielhaft einen möglichen Messkurvenverlauf bei Aufnahme von Druckimpulsen und Bohrwiderstandsmessung bei einer Untersuchung eines Baumstammes, wenn die Bohrnadel aus gesundem Holz auf Moderfäule trifft.
**Fig. 3c** zeigt beispielhaft einen möglichen Messkurvenverlauf bei Aufnahme von Druckimpulsen und Bohrwiderstandsmessung bei einer Untersuchung eines Baumstammes, wenn die Bohrnadel aus gesundem Holz auf Weißfäule trifft.
**Fig. 4** zeigt in schematischer Darstellung einen Bediener mit einer erfindungsgemäßen Untersuchungsvorrichtung aus mobiler Recheneinheit und Bohrgerät bei der erfindungsgemäßen gesteuerten Untersuchung eines Holzstrommasts.
**Fig. 5** zeigt in schematischer Draufsicht einen Tablet-PC als mobile Recheneinheit.
**Fig. 6** zeigt in schematischer Darstellung den Holzstrommast aus Fig. 4 mit den zur Durchführung des erfindungsgemäßen Verfahrens gemäß einer Ausführungsform vorgegebenen Bohrungen.
**Fig. 7** zeigt beispielhaft einen Messkurvenverlauf einer Bohrwiderstandsmessung bei einer Untersuchung eines Holzmasts mit gesundem Holz.
**Fig. 8** zeigt vereinfachte Messkurvenverläufe der wie in Fig. 6 vorgegeben durchgeführten Bohrwiderstandsmessungen I bis III bei einer Untersuchung eines Holzmasts mit gesundem Holz.
**Fig. 9** zeigt vereinfachte Messkurvenverläufe der wie in Fig. 6 vorgegeben zunächst durchgeführten Bohrwiderstandsmessungen I bis III bei einer Untersuchung eines Holzmasts mit Fäule, und den in Folge dessen weiteren vorgegebenen Messungen IV bis VI.

Zur Durchführung des erfindungsgemäßen Verfahrens wird ein kombiniertes Bohrwiderstands- und Impulslaufzeitmessverfahren durchgeführt, wie es aus der DE 10 2009 013 069 bekannt ist, auf die hiermit vollumfänglich Bezug genommen wird.

Das erfindungsgemäße Verfahren lässt sich besonders präzise ausführen, wenn die untersuchten Körper zylindrisch, näherungsweise zylindrisch oder säulenförmig sind, wobei unter einer Säulenform ein längliches geometrisches Element definierter Geometrie verstanden wird, wie beispielsweise ein Konus oder auch eine Säule von rechteckiger, quadratischer oder polyedrischer Grundfläche. Auch konische Säulen mit elliptischer Grundfläche lassen sich gut heranziehen um das erfindungsgemäße Verfahren auszuführen.

Um das erfindungsgemäße Verfahren zur gesteuerten Beschaffenheitsuntersuchung auszuführen, wird zur Durchführung der kombinierten Bohrwiderstands- und Impulslaufzeitmessung eine Untersuchungsvorrichtung verwendet, die neben einem Bohrgerät mit einer Bohrnadel und Sensoren zur Impulsaufnahme sowie Mitteln zum Erfassen und gegebenenfalls Aufzeichnen der durch die Bohrnadel erzeugten oder aufgenommenen Signale eine Standort-Bestimmungseinrichtung wie einen GPS-Empfänger und eine mobile Recheneinheit wie einen Tablet-PC umfasst. Diese mobile Recheneinheit ist zur Durchführung des erfindungsgemäßen Verfahrens ausgebildet und weist eine Kommunikationsschnittstelle zum Aufbau einer Datenübertragungsverbindung mit einer entsprechenden Kommunikationsschnittstelle der Erfassungsmittel für die durch die Bohrnadel erzeugten oder aufgenommenen Signale.

Die Kommunikationsverbindung zwischen den Erfassungsmitteln und der mobilen Recheneinheit, um die erfassten und gegebenenfalls aufgezeichneten durch die Bohrnadel erzeugten oder aufgenommenen Signale zu übertragen, kann verdrahtet oder drahtlos beispielsweise über ein lokales Funkverbindungsnetz (WLAN) wie Bluetooth® oder Wi-Fi, oder ähnliches erfolgen.

In einem ersten Schritt werden mit der mobilen Recheneinheit Kennzeichnungsdaten eines zu untersuchenden Objekts erfasst. Diese Kennzeichnungsdaten können der Standort als Ortsbeschreibung, eine Objektbezeichnung wie z. B. eine Mast-nummer und/oder geografische Koordinaten des Objekts, die mittels der Standort-Bestimmungseinrichtung ermittelt werden, sein. Die Standort-Bestimmungseinrichtung kann ferner helfen, ein zu untersuchendes Objekt aufzufinden.

Sind die Kennzeichnungsdaten des zu untersuchenden Objekts mit der mobilen Recheneinheit erfasst, so bestimmt diese einen Auswertungsvorgang für das zu untersuchende Objekt, wobei sie die Art und Anzahl der an dem zu untersuchenden Objekt durchzuführenden kombinierten Bohrwiderstands- und Impulslaufzeitmessungen vorgibt. Durch den Bediener werden dann die für das Objekt vorgegebenen Messungen mit dem Bohrgerät ausgeführt, wobei die dabei erhaltenen Impulslaufzeiten und Bohrwiderstandswerte von den Erfassungsmitteln über die Kommunikationsverbindung einer Datenverarbeitungssoftware der mobilen Recheneinheit zugeführt werden.

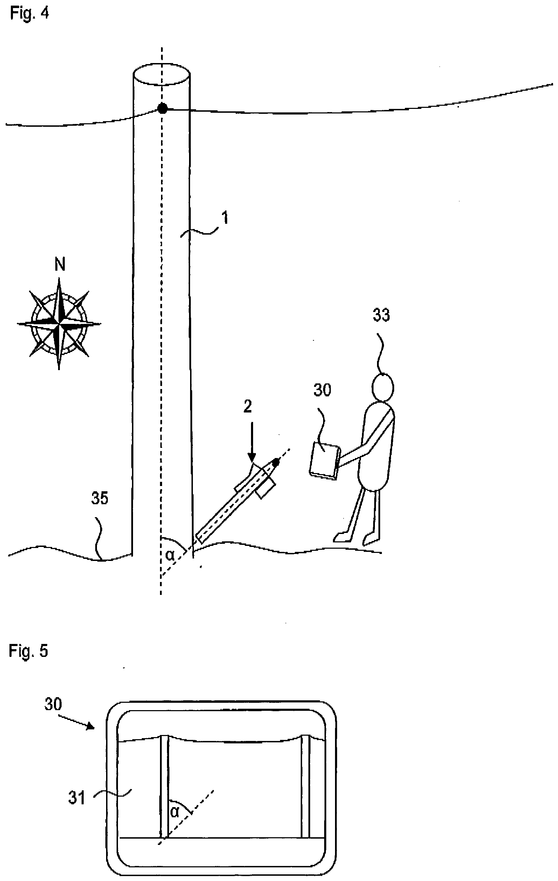

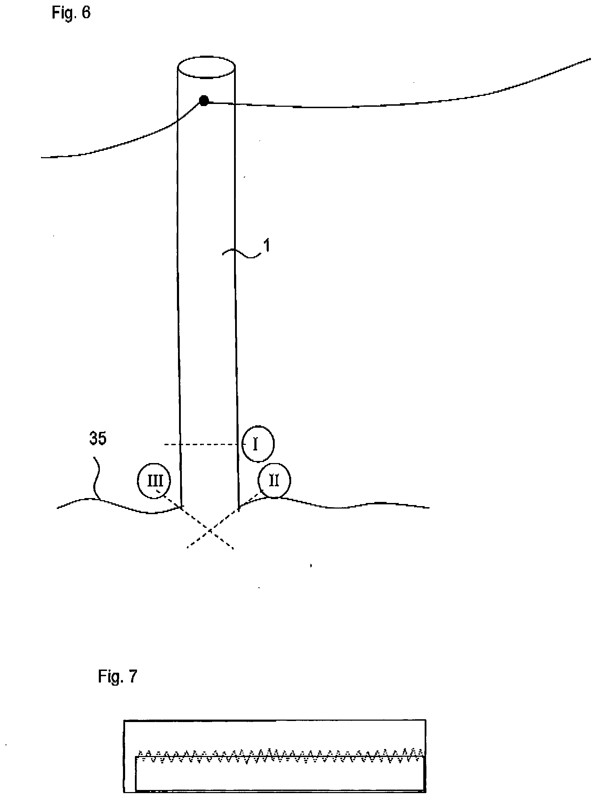

Ein Beispiel zum Einsatz des erfindungsgemäßen gesteuerten Beschaffenheitsuntersuchungsverfahren ist die Holzmastenkontrolle, bei der das zu untersuchende Objekt 1 ein Holzmast 1, dargestellt in **Fig. 4** **und** **6**, ist. Der Bediener 33 ermittelt am Standort des Masts 1 die GPS-Daten mittels der in der mobilen Recheneinheit 30 oder dem Bohrgerät 2 integrierten Standort-Bestimmungseinrichtung und nimmt alle Daten am Mast 1 auf. Zu diesen Daten können neben den GPS-Daten auch eine Standortbezeichnung und eine Mast-Nummer gehören. Weiter kann dazu eine visuelle Mastkontrolle mit entsprechender Eingabe der optischen Eindrücke in die Recheneinheit 30 gehören, sowie die Aufnahme eines Fotos zur Dokumentation des untersuchten Mastes 1. Hierfür kann beispielsweise eine in die mobile Recheneinheit 30 integrierte Kamera genutzt werden. Alternativ kann ein mit einer Digitalkamera oder einem Smartphone aufgenommenes Foto des Masts 1 mittels Kabel oder drahtlos auf die mobile Recheneinheit 30 überspielt werden.

Das auf der Recheneinheit 30 installierte Auswerteprogramm enthält die Punkte des durchzuführenden Auswertungsvorgangs, in dem die durchzuführenden Messungen mit Vorgaben und Reihenfolge enthalten sind. Der Bediener 33 kann dann, nach Erfassung der kennzeichnenden Daten des Masts 1, entsprechend der Anweisungen der Recheneinheit 30 die Messungen an dem erfassten Mast 1 durchführen. Die Vorgaben zu den Messungen können z. B. eine Messhöhe über dem Grund 35 sowie einen Einführungswinkel α enthalten. **Fig. 5** zeigt einen Tablet-PC 30 als Recheneinheit mit einem Bildschirm 31, auf dem der Einführungswinkel α der nächsten, an dem Mast 1 durchzuführenden Messung angezeigt wird. Weitere Anweisungen nach erfolgter Messauswertung können dann ebenfalls über die Recheneinheit 30 erfolgen.

Um die Messbohrung mit dem Bohrgerät 2 möglichst präzise ausführen zu können, kann an die Steuerungselektronik des Bohrgeräts 2 eine Winkelanzeige angebunden sein, um den von der Recheneinheit 30 vorgegebenen Bohrwinkel α einzuhalten. Ferner umfasst das Bohrgerät 2 einen Kompass, um eine hinsichtlich Himmelsrichtung vorgegebene Bohrstelle an dem Mast 1 aufzufinden. Zusätzlich gestattet der mit der Steuerungselektronik des Bohrgeräts 2 verknüpfte Kompass, die Himmelsrichtung zu erfassen, in der das Bohrgerät 2 in Bezug zu dem Mast 1 bei Durchführung einer Messung ausgerichtet ist, und bei der Auswertung nach Übertragung der Daten an die mobile Recheneinheit 30 zu berücksichtigen.

Die Steuerungselektronik stellt somit eine Art "Fernsteuerung" bereit, die zulässt, dass das Bohrgerät 2 über die mobile Recheneinheit 30, die ein externer PC wie ein Notebook, Laptop oder Netbook oder auch ein Tablet-PC wie ein iPad® oder Smartphone sein kann, gesteuert wird. Mittels dieser Steuerung können dem Bediener verschiedene Arbeitsabfolgen vorgegeben werden, die er mit dem Bohrgerät 2 auszuführen hat.

Bei der Untersuchung wird der Bediener 33 aufgefordert, eine erste Messung I (siehe **Fig. 6**) unter 90 Grad in den Mast 1 zu bohren, um die Holzdichte zu ermitteln. Danach erfolgt im Bereich der Erdgleiche 35 eine zweite und/oder dritte Messung II, III unter einem vorgegebenen Einführungswinkel α, der z. B. 30 oder 45 Grad betragen kann.

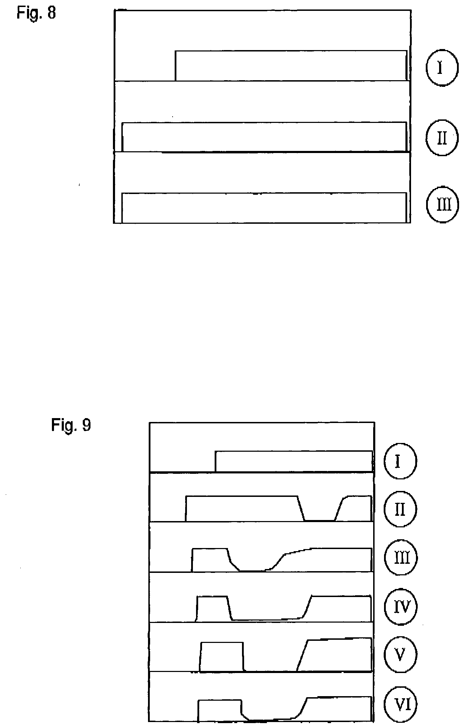

**Fig. 7** zeigt eine Sollkurve der mittels Bohrwiderstandsmessung aufgenommenen Holzdichte, gemessen an einem intakten (Vergleichs-) Holzmast. Die Messwerte der gepunkteten Linie schwanken um einen Mittelwert, der durch die durchgezogene Gerade angezeigt ist. In den in **Fig. 8 und 9** gezeigten Messkurven der Holzdichte sind nur die gemittelten Verlaufslinien dargestellt. **Fig. 8** zeigt die an einem Mast 1 aufgenommenen Messkurven bei der 90° Messung I senkrecht in den Mast 1, und der unter einem von 90° abweichenden nach unten weisenden Einführwinkel α durchgeführten zweiten und dritten Messungen II, III im Bereich der Erdgleiche 35. Die Messkurvenverläufe zeigen hierbei eine gleichmäßige Holzdichte und damit einen intakten Mast an.

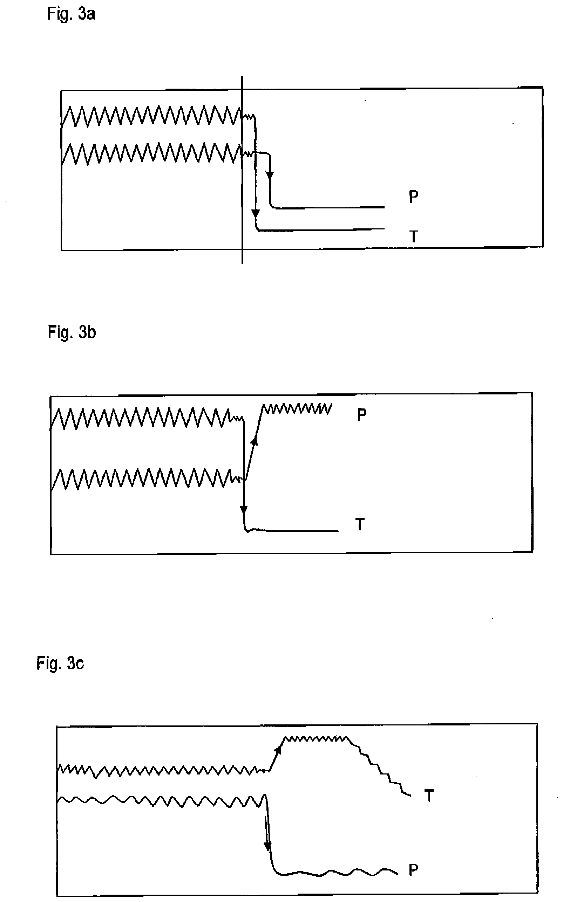

Ergibt sich bei einer oder mehreren der zunächst vorgegebenen Messungen I, II, III eine Abweichung der Holzdichte, wie in **Fig. 9** zu sehen, und es wird damit bestehende Fäule ermittelt, kann der Bediener von der Recheneinheit 30 aufgefordert werden, weitere, im vorliegenden Beispiel drei Messungen IV, V, VI durchzuführen. Auch hier können ihm Art, d. h. Höhe über dem Boden und Einführwinkel und Anzahl der weiteren Messungen vorgegeben werden. Der Übersichtlichkeit wegen sind in **Fig. 7** sowie in **Fig. 8 und 9** nur die Bohrwiderstandsmesskurven dargestellt. Selbstverständlich werden bei den vorgegebenen Messungen I, II, III und den gegebenenfalls weiteren Messungen IV, V, VI neben dem Bohrwiderstand auch die Impulslaufzeiten berücksichtigt, wie nachfolgend in Zusammenhang mit **Fig. 3a**, **3b und 3c** noch beschrieben wird.

So wird nach Durchführung der ersten Messungen I, II, III von der Recheneinheit 30 in Abhängigkeit der Messergebnisse entschieden, ob weitere Messungen durchzuführen sind. Das von der Recheneinheit 30 ausgeführte Softwareprogramm zur Auswertung der Messergebnisse errechnet mit den erhaltenen Messwerten automatisch die Fäule, und durch Vergleich mit Vorgabewerten kann eine Beurteilung des Mastes 1 durchgeführt werden. Kommt die Recheneinheit 30 nach Auswertung der Messungen zu dem Ergebnis, dass der Mast 1 im Stand gefährdet ist, so kann der Mast 1 in der Recheneinheit 30 als defekt und zum Austausch ausgeschrieben gelistet werden; ferner kann mit der Recheneinheit 30 eine Anweisung an den Bediener 33 ausgegeben werden, den defekten Mast 1 zu kennzeichnen. In ähnlicher Weise kann vorgesehen sein, auch als intakt erkannte Masten mit einem Kennzeichen zu versehen, um sichtbar zu machen, dass dieser Mast kontrolliert und für in Ordnung befunden wurde.

Zur Durchführung der Messungen mit dem kombinierten Bohrwiderstands- und Impulslaufzeitmessverfahren werden zunächst wenigstens zwei, vorzugsweise eine Vielzahl von Sensoren zur Aufnahme von Drucksignalen in einer oder mehreren Ebenen an dem Umfang des zu untersuchenden, säulenförmigen oder zylindrischen Abschnitts des Körpers angeordnet.

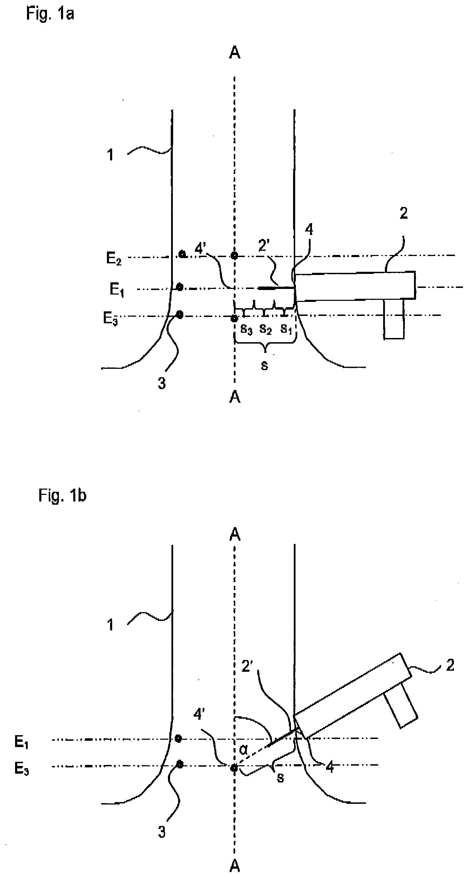

**Fig. 1a** zeigt beispielhaft die Ausführung des Messverfahrens an einem Baumstamm 1, an dessen Umfang eine Vielzahl von Sensoren 3 in einem Schritt a) angeordnet werden, wobei drei Reihen von Sensoren 3 in horizontalen Ebenen E₁, E₂, E₃ angeordnet zu sehen sind, die ergänzt werden von weiteren Sensoren 3 an der nicht zu sehenden Seite des Baumstamms 1. Die Bohrnadel 2' des an dem Baumstamm 1 angesetzten Bohrgeräts 2 wird von einer Einführstelle 4 diskontinuierlich bis zu einer vorbestimmten Eindringtiefe radial in Richtung der Längsachse A-A des zu untersuchenden Abschnitts in den Baum 1 eingeführt. Die Durchführung dieses Schrittes b) erfolgt an einem Baum auf völlig analoge Weise wie an einem Holzmast oder einer Beton- oder Steinsäule.

Das diskontinuierliche Einführen der Bohrnadel 2' in Schritt b) umfasst das Impuls induzierende Anhalten und wieder Anfahren der Bohrnadel 2', nachdem die Bohrnadel 2' einen Streckenabschnitt s₁ auf der Strecke s zwischen der Einführstelle 4 der Bohrnadel 2' am Umfang des Körpers 1 und einer Stelle 4' der vorbestimmten Einführtiefe zurückgelegt hat. Der Vortrieb der Nadel 2' wird an dieser Stelle unterbrochen, die Nadel 2' kommt zur Ruhe und der Vortrieb wird erneut eingeschaltet. Durch dieses Anhalten der Bohrnadel 2' und erneutes Aufnehmen des Vortreibens wird der gewünschte Impuls im Inneren des zu untersuchenden Baumes induziert. Auf diese Weise werden die weiteren Streckenabschnitte s₂ und s₃ zurückgelegt. Die Eindringtiefe bei dem erfindungsgemäßen Bohrwiderstands-Impulsmessverfahren hängt dabei letztlich von der Länge der Bohrnadel 2' ab, die gegebenenfalls eine Länge haben kann, die bis zur Längsachse A-A des zu untersuchenden Objekts reichen kann.

**Fig. 1b** zeigt alternativ eine Ausführung des Messverfahrens, bei dem die Bohrnadel 2' unter einem vorgegebenen Winkel α nach unten geneigt in den Baumstamm 1 eingeführt wird. Im vorliegenden Beispiel beträgt der Einführwinkel α zwischen Bohrachse und der Längsachse A-A des zu untersuchenden Abschnitts des Baumstamms 1 45°. Indem die Impulse in Richtung der Baumwurzel gelenkt werden, ist es möglich, insbesondere von der Wurzel her kommende Fäulnis zu erfassen. Die Sensoren 3 werden entsprechend im unteren Baumstammbereich angeordnet. Entsprechendes gilt für die in Zusammenhang mit **Fig. 4** **und** **6** beschriebene Messung an dem Holzmast 1. Auch dort wird vor allem der im Bodenbereich verankerte Abschnitt des Masts 1 überprüft, der infolge der dort herrschenden Feuchtigkeit vermehrt fäulnisgefährdet ist. Bei der Auswertung der Messergebnisse aus Impulslaufzeitmessung und Bohrwiderstandsmessung wird der Winkel α rechnerisch berücksichtigt.

Durch das Anhalten und wieder Anfahren der Nadel 2' breiten sich Impulswellen vom Ort ihrer Erzeugung kugelförmig nach außen fort, und überall dort, wo Sensoren 3 angeordnet sind, kann der ankommende Druckimpuls detektiert werden. Wenn eine Anzahl von Sensoren 3 auf drei Ebenen E₁, E₂, E₃ verteilt ist, wie in **Fig. 1a** am Umfang 1 des Baumes gezeigt, so können die ankommenden Druckimpulse in drei Ebenen E₁, E₂, E₃ gemessen werden. Damit erfolgt eine Messwerterfassung in drei unterschiedlichen Höhen des Baumstamms 1, obgleich das Einführen der Bohrnadel 2' lediglich entlang einer Strecke s ausgeführt wird. Natürlich kann die Nadel 2' dann zur ergänzenden Messwerterfassung und zur Erhöhung der Genauigkeit auch in den weiteren Ebenen an einer Einführstelle angesetzt und eingeführt werden.

Die Vorteile der kombinierten Bohrwiderstands- und Impulslaufzeitmessung als invasives Verfahren liegen in der Minimierung der schädigenden Wirkung im Vergleich zu anderen Verfahren und einer präziseren Messwerterfassung. Während des Vortriebs entlang der Streckenabschnitte s₁, s₂, s₃ in Richtung der Längsachse A-A des Körpers werden Bohrwiderstandsmessungen über den jeweiligen Streckenabschnitt s₁, s₂, s₃ ausgeführt und gleichzeitig Drucksignale durch die Sensoren zur Impulslaufzeitmessung aufgenommen, so dass durch das diskontinuierliche Einführen der Bohrnadel 2' zeitsparend zwei auf unterschiedlichen physikalischen Parametern basierende Messwertdatensätze, ein Bohrwiderstandsdiagramm und ein Impulstomogramm, erhalten werden.

Durch eine Korrelation der Bohrwiderstandswerte und der Impulslaufzeitdaten kann ein Datensatz erhalten werden, der es ermöglicht, eine eventuelle Schädigung des Materials in kürzest möglicher Messzeit aufzunehmen und eine präzise Lokalisierung durchzuführen. Darüber hinaus ergibt die Korrelation der auf den unterschiedlichen Parametern basierenden Messwerte eine Aussage über die Art der Schädigung. Dies ist beispielsweise bei der Untersuchung von Holz relevant, wenn festgestellt werden soll, ob das Holz unter einer Fäulnis leidet oder ob lediglich auf Grund bestimmter Wachstumsbedingungen sehr weiches Holz, das jedoch gesund ist, vorliegt.

Unterschiedliche Fäulnisarten führen entsprechend zu unterschiedlichen Resultaten: So zeigt **Fig. 3a** beispielhaft einen möglichen Messkurvenverlauf bei Aufnahme von Druckimpulsen P und gleichzeitig ausgeführter Bohrwiderstandsmessung T bei einer Untersuchung eines Baumstammes, der unter Braunfäule leidet, wie sie etwa durch den Schwefelporling hervorgerufen wird, wenn die Bohrnadel aus dem gesundem Holz in das braunfaule Holz einbricht. Die durch Druckimpulse P erhaltenen Signale zeigen einen Verlauf, der dem der Bohrwiderstandsmessung T entspricht, während bei einer Moderfäule, die durch einen Brandkrustenpilz erzeugt werden kann, siehe **Fig. 3b**, der Verlauf der Bohrwiderstandsmess-Signale T sich wie bei der Braunfäule verhält, wohingegen die Druckimpuls-Signale P sich gegenläufig verhalten. Damit lassen sich diese beiden Fäulnisarten unterscheiden.

**In** **Fig. 3c** zeigt sich, dass der Bohrwiderstand T bei der aufgetretenen Weißfäule sprunghaft ansteigt, und nach Erreichen eines Plateaus wieder abfällt, wohingegen die Druckimpulswerte P beim Übergang in das weißfaule Holz sprunghaft abfallen.

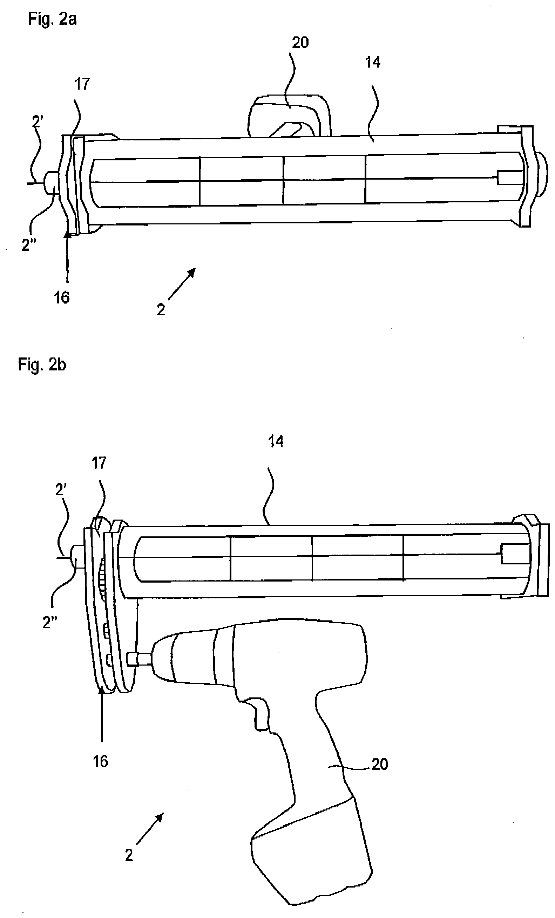

Das Bohrgerät 2, wie es in **Fig. 2a und 2b** beispielhaft dargestellt ist, und das mit der Bohrnadel 2' bestückt ist, kann einen mit einem Antriebsaggregat 20, das eine handelsübliche Handbohrmaschine sein kann, operativ gekoppelten Bohraufsatz 16 aufweisen, der in einem Gehäuse oder zumindest, wie vorliegend gezeigt, zwischen zwei Halteplatten ein Getriebe 17 enthält. Das Getriebe 17 - das ein Planetengetriebe sein kann - überträgt das durch das Antriebsaggregat 20 erzeugte Drehmoment über die Bohrnadel-Aufnahmevorrichtung 2" auf die Bohrnadel 2'. Durch den Bohraufsatz 16 können die Mittel 14 zum Erfassen und gegebenenfalls Aufzeichnen der von durch die Bohrnadel 2' erzeugten oder aufgenommenen Signale an das Bohrgerät 2 angebunden werden, wobei die Erfassungsmittel 14 eine die Bohrnadel 2' stabilisierende und führende Haltevorrichtung umfassen, die mit dem Bohraufsatz 16 verbunden ist. Im Übrigen können die Erfassungsmittel 14 auch die aus der Impulslaufzeitmessung resultierenden und von den Sensoren detektierten Signale aufzeichnen.

Die zu der Gesamtvorrichtung zur Ausführung des Messverfahrens gehörenden Sensoren sind in **Fig. 2a und 2b** nicht gezeigt.

Die so geführte und stabilisierte Bohrnadel ist geeignet, mit dem Bohrgerät und der das Bohrgerät umfassenden Vorrichtung das erfindungsgemäße Verfahren auszuführen, das im Vorliegenden zwar an Holzobjekten (Holzmast und Baumstamm) beispielhaft dargelegt ist, sich aber auch zur Überprüfung von Objekten aus anderem Material wie beispielsweise Beton eignet. So kann etwa auch ein Betonmast untersucht werden, wie er beispielsweise auch als Elektromast verwendet wird. Wenn ein solcher Mast aus Herstellungsgründen innen hohl ist, neigt er dazu, von innen heraus zu korrodieren, etwa, wenn im Inneren des Mastes Feuchtigkeit durch Kapillarkräfte in die Höhe gezogen wird. Zersetzungsmechanismen des Betons setzen ein und Korrosionsstellen bilden sich.

Derartige Zersetzungsmechanismen kommen auch in natürlichen Materialien vor, wie beispielsweise bei Sandstein. Eine Vielzahl natürlicher Materialien ist derartigen Zersetzungsvorgängen ausgesetzt, ob sie nun als röhrenförmige oder zumindest innen teilweise hohle Objekte oder als Massivobjekte eingesetzt werden. Auch hier ist es überaus vorteilhaft, wenn das Untersuchungsverfahren schnell, reproduzierbar und präzise ohne Schädigung des Materials durch das Untersuchungsverfahren selbst ausgeführt werden kann, daher ist das erfindungsgemäße Verfahren besonders geeignet zur Untersuchung solcher Baukörper. Die Nadel wird dabei in den Randbereich des Objekts eingeführt und es werden Impulse induziert. Völlig analog wie bei der Untersuchung des Baumes weisen die Ausbreitungsgeschwindigkeiten der Impulswellen unterschiedliche Laufzeiten auf, wenn das Material korrodiert, also geschädigt ist, und daher hohl, porös oder auf andere Weise instabil ist, wodurch, wenn keine ausreichende Randschicht mehr vorhanden ist, um den Körper zuverlässig zu stabilisieren, der Körper eine Verkehrsgefährdung darstellen kann. Durch Heranziehen eines Vergleichsobjekts als Kalibriergegenstand kann sehr schnell der Zustand des Materials geprüft und Schädigungen festgestellt werden. Der Fachmann weiß, dass das Bereitstellen von Vergleichsmesswerten, das Vergleichen von Vergleichsmesswerten und das Ermitteln einer Schädigung des Untersuchungsobjekts durch Differenzbetrachtung zwischen Messobjekt und Kalibrierobjekt mittels einer elektronischen Auswertung erfolgen kann. Ferner ist dem Fachmann bekannt, wie er eine dreidimensionale Darstellung der Messwerte erzielt.

**BEZUGSZEICHENLISTE**

| | |
|---|---|
| 1 | Objekt, Baum, Mast |
| 2 | Bohrgerät |
| 2' | Bohrnadel |
| 3 | Sensoren zur Impulsdetektion |
| 4 | Einführstelle |
| 4' | Stelle der vorbest. Einführtiefe |
| 5 | Impulswellen |
| 6 | Korrosionsstellen |
| 10 | Säule |
| 14 | Signalerfassungsmittel, bzw. Aufzeichnungs- und Führungsmittel |
| 16 | Bohraufsatz |
| 17 | Getriebe |
| 20 | Antriebsaggregat |
| 30 | Recheneinheit, Tablet-PC |
| 31 | Bildschirm der mobilen Recheneinheit |
| 33 | Bediener |
| 35 | Erdboden, Bodengleiche |
| A-A | Längsachse des Objekts |
| E₁, E₂, E₃, | horizontale Ebene |
| S, S₁ | Strecke, Streckenabschnitt |
| α | Einführwinkel |

gerufen wird, wenn die Bohrnadel aus dem gesundem Holz in das braunfaule Holz einbricht. Die durch Druckimpulse P erhaltenen Signale zeigen einen Verlauf, der dem der Bohrwiderstandsmessung T entspricht, während bei einer Moderfäule, die durch einen Brandkrustenpilz erzeugt werden kann, siehe **Fig. 3b**, der Verlauf der Bohrwiderstandsmess-Signale T sich wie bei der Braunfäule verhält, wohingegen die Druckimpuls-Signale P sich gegenläufig verhalten. Damit lassen sich diese beiden Fäulnisarten unterscheiden.

In **Fig. 3c** zeigt sich, dass der Bohrwiderstand T bei der aufgetretenen Weißfäule sprunghaft ansteigt, und nach Erreichen eines Plateaus wieder abfällt, wohingegen die Druckimpulswerte P beim Übergang in das weißfaule Holz sprunghaft abfallen.

Das Bohrgerät 2, wie es in **Fig. 2a und 2b** beispielhaft dargestellt ist, und das mit der Bohrnadel 2' bestückt ist, kann einen mit einem Antriebsaggregat 20, das eine handelsübliche Handbohrmaschine sein kann, operativ gekoppelten Bohraufsatz 16 aufweisen, der in einem Gehäuse oder zumindest, wie vorliegend gezeigt, zwischen zwei Halteplatten ein Getriebe 17 enthält. Das Getriebe 17 - das ein Planetengetriebe sein kann - überträgt das durch das Antriebsaggregat 20 erzeugte Drehmoment über die Bohrnadel-Aufnahmevorrichtung 2" auf die Bohrnadel 2'. Durch den Bohraufsatz 16 können die Mittel 14 zum Erfassen und gegebenenfalls Aufzeichnen der von durch die Bohrnadel 2' erzeugten oder aufgenommenen Signale an das Bohrgerät 2 angebunden werden, wobei die Erfassungsmittel 14 eine die Bohrnadel 2' stabilisierende und führende Haltevorrichtung umfassen, die mit dem Bohraufsatz 16 verbunden ist. Im Übrigen können die Erfassungsmittel 14 auch die aus der Impulslaufzeitmessung resultierenden und von den Sensoren detektierten Signale aufzeichnen.

Die zu der Gesamtvorrichtung zur Ausführung des Messverfahrens gehörenden Sensoren sind in **Fig. 2a und 2b** nicht gezeigt.

Die so geführte und stabilisierte Bohrnadel ist geeignet, mit dem Bohrgerät und der das Bohrgerät umfassenden Vorrichtung das erfindungsgemäße Verfahren auszuführen, das im Vorliegenden zwar an Holzobjekten (Holzmast und Baumstamm) beispielhaft dargelegt ist, sich aber auch zur Überprüfung von Objekten aus anderem Material wie beispielsweise Beton eignet. So kann etwa auch ein Betonmast untersucht werden, wie er beispielsweise auch als Elektromast verwendet wird. Wenn ein solcher Mast aus Herstellungsgründen innen hohl ist, neigt er dazu, von innen heraus zu korrodieren, etwa, wenn im Inneren des Mastes Feuchtigkeit durch Kapillarkräfte in die Höhe gezogen wird. Zersetzungsmechanismen des Betons setzen ein und Korrosionsstellen bilden sich.

Derartige Zersetzungsmechanismen kommen auch in natürlichen Materialien vor, wie beispielsweise bei Sandstein. Eine Vielzahl natürlicher Materialien ist derartigen Zersetzungsvorgängen ausgesetzt, ob sie nun als röhrenförmige oder zumindest innen teilweise hohle Objekte oder als Massivobjekte eingesetzt werden. Auch hier ist es überaus vorteilhaft, wenn das Untersuchungsverfahren schnell, reproduzierbar und präzise ohne Schädigung des Materials durch das Untersuchungsverfahren selbst ausgeführt werden kann, daher ist das erfindungsgemäße Verfahren besonders geeignet zur Untersuchung solcher Baukörper. Die Nadel wird dabei in den Randbereich des Objekts eingeführt und es werden Impulse induziert. Völlig analog wie bei der Untersuchung des Baumes weisen die Ausbreitungsgeschwindigkeiten der Impulswellen unterschiedliche Laufzeiten auf, wenn das Material korrodiert, also geschädigt ist, und daher hohl, porös oder auf andere Weise instabil ist, wodurch, wenn keine ausreichende Randschicht mehr vorhanden ist, um den Körper zuverlässig zu stabilisieren, der Körper eine Verkehrsgefährdung darstellen kann. Durch Heranziehen eines Vergleichsobjekts als Kalibriergegenstand kann sehr schnell der Zustand des Materials geprüft und Schädigungen festgestellt werden. Der Fachmann weiß, dass das Bereitstellen von Vergleichsmesswerten, das Vergleichen von Vergleichsmesswerten und das Ermitteln einer Schädigung des Untersuchungsobjekts durch Differenzbetrachtung zwischen Messobjekt und Kalibrierobjekt mittels einer elektronischen Auswertung erfolgen kann. Ferner ist dem Fachmann bekannt, wie er eine dreidimensionale Darstellung der Messwerte erzielt.

**BEZUGSZEICHENLISTE**

| | |
|---|---|
| 1 | Objekt, Baum, Mast |
| 2 | Bohrgerät |
| 2' | Bohrnadel |
| 3 | Sensoren zur Impulsdetektion |
| 4 | Einführstelle |
| 4' | Stelle der vorbest. Einführtiefe |
| 5 | Impulswellen |
| 6 | Korrosionsstellen |
| 10 | Säule |
| 14 | Aufzeichnungs- und Führungsmittel |
| 16 | Bohraufsatz |
| 17 | Getriebe |
| 20 | Antriebsaggregat |
| 30 | Recheneinheit, Tablet-PC |
| 31 | Bildschirm der mobilen Recheneinheit |
| 33 | Bediener |
| 35 | Erdboden, Bodengleiche |
| A-A | Längsachse des Objekts |
| E₁, E₂, E₃, | horizontale Ebene |
| S, S₁ | Strecke, Streckenabschnitt |
| α | Einführwinkel |

## Patentansprüche

1. Vorrichtung zur Durchführung eines Beschaffenheitsuntersuchungsverfahrens von säulenförmigen oder zylindrischen Objekten (1), die
- ein Bohrgerät (2) mit einer Bohrnadel (2'),
- Signalerfassungsmittel (14) für Bohrwiderstandsmess- und Impulslaufzeitmess-Signale, die durch die in ein zu untersuchendes Objekt (1) einführbare Bohrnadel (2') erzeugbar oder aufnehmbar sind,
wobei die Signalerfassungsmittel (14) für die Impulslaufzeitmess-Signale mehrere Drucksensoren (3) aufweisen, die zum Anordnen an dem Objekt (1) ausgebildet sind,
aufweist,
**dadurch gekennzeichnet, dass**
das Bohrgerät (2)
- eine Standort-Bestimmungseinrichtung,
- einen Kompass und
- eine Anzeigevorrichtung für einen Einführwinkel (α) einer durchzuführenden Bohrwiderstands- und Impulslaufzeitmessung aufweist,
und eine getrennt von dem Bohrgerät (2) handhabbare Recheneinheit (30) umfasst, wobei
die Standort-Bestimmungseinrichtung, der Kompass, die Anzeigevorrichtung für den Einführwinkel (α) und/oder die Mittel (14) mit einer Steuerelektronik des Bohrgeräts (2) operativ gekoppelt sind, und
wobei die Recheneinheit (30) und die Signalerfassungsmittel (14) jeweils eine Kommunikationsschnittstelle zum Aufbau einer Kommunikationsverbindung zur Übertragung der durch die Bohrnadel (2') erzeugten oder aufgenommenen Signale von den Signalerfassungsmitteln (14) an die Recheneinheit (30) aufweisen, wobei
die Recheneinheit (30)
- zumindest eine Schnittstelle zur Eingabe von Kennzeichnungsdaten des zu untersuchenden Objekts (1), die zumindest einen durch die Standort-Bestimmungseinrichtung bestimmbaren Standort des Objekt (1) umfassen,
- eine Prozessor- und Speichereinheit mit einem Datenverarbeitungsprogramm konfiguriert zur Verarbeitung der Kennzeichnungsdaten des zu untersuchenden Objekts (1) und zum Vorgeben eines Untersuchungsmodus für das zu untersuchende Objekt (1) an einen Benutzer und zum Auswerten der durch die Bohrnadel (2') erzeugten oder aufgenommenen Signale, wobei der Untersuchungsmodus die Vorgabe einer Art, wie die Messungen durchzuführen sind, umfasst, und
- eine Ausgabeeinheit für die Ausgabe des vorgegebenen Untersuchungsmodus für das zu untersuchende Objekt (1) und zur Ausgabe von Auswertungsergebnissen an den Benutzer aufweist.

2. Vorrichtung nach Anspruch 1,
**dadurch gekennzeichnet, dass**
die Kommunikationsschnittstelle eine Funkschnittstelle zum Aufbau der Datenübertragungsverbindung über ein lokales Funknetz ist.

3. Vorrichtung nach Anspruch 1 oder 2,
**dadurch gekennzeichnet, dass**
die Standort-Bestimmungseinrichtung, einen GPS-Empfänger aufweist.

4. Vorrichtung nach zumindest einem der Ansprüche 1 bis 3,
**dadurch gekennzeichnet, dass**
die Vorrichtung eine Kamera umfasst, und
die Recheneinheit (30) ein Notebook, Smartphone, Tablet-PCs, Netbooks, i-Pad ist.

5. Beschaffenheitsuntersuchungsverfahren von säulenförmigen oder zylindrischen Objekten (1) unter Verwendung der Vorrichtung nach einem der Ansprüche 1 bis 4,
**umfassend die Schritte**
A) Ermitteln von Kennzeichnungsdaten, zumindest eines Standorts des Objekts (1), mit der Standortbestimmungseinheit und Erfassen der Daten durch die Recheneinheit (30),
B) einem Benutzer mit der Ausgabeeinheit Ausgeben eines Untersuchungsmodus für das zu untersuchenden Objekt (1), dabei Vorgeben zumindest einer Bohrwiderstands- und Impulslaufzeitmessweise, einer Messhöhe über der Erdgleiche (35) und einen Einführwinkel (α), und einer Mindestanzahl der an dem zu untersuchenden Objekt (1) durchzuführender Messungen, und
C) Durchführen der Untersuchung gemäß dem vorgegebenen Untersuchungsmodus und Zuführen der aufgenommenen Messdaten, die zumindest Bohrwiderstands- und Impulslaufzeitmessdaten sind, dem Datenverarbeitungsprogramm der Recheneinheit (30) und Auswerten derselben und
D) dem Benutzer Ausgeben des Auswertungsergebnisses.

6. Verfahren nach Anspruch 5,
**umfassend die Schritte**
E) in Abhängigkeit des Auswertungsergebnisses durch die Recheneinheit (30) auswählen, ob die Untersuchung an dem Objekt (1) fortzuführen ist, falls ja
F) Vorgeben des Untersuchungsmodus und Ausführen der Schritte C) und D).

7. Verfahren nach zumindest einem der Ansprüche 5 oder 6,
wobei die aufgenommenen Messdaten Bildaufnahmedaten umfassen.

8. Verfahren nach zumindest einem der Ansprüche 5 bis 7,
wobei die Durchführung der Bohrwiderstands- und Impulslaufzeitmessung umfasst:
a) Anordnen der Sensoren (3) zur Drucksignalaufnahme in zumindest einer Ebene (E1, E2, E3) an einem Umfang des säulenförmigen oder zylindrischen Objekts (1),
b) das diskontinuierliche Einführen der Bohrnadel (2') des Bohrgerätes (2) unter Durchführung der Bohrwiderstandsmessung bis zu einer vorbestimmten Eindringtiefe radial in Richtung einer Längsachse (A-A) des Abschnitts in den säulenförmigen oder zylindrischen Abschnitt des Objekts (1), wobei das diskontinuierliche Einführen ein Impuls induzierendes Anhalten, gefolgt von einem Wiederanfahren der Bohrnadel (2') nach Zurücklegen eines oder mehrerer Streckenabschnitte (s₁,s₂,s₃) auf einer Strecke (s) zwischen einer Einführstelle (4) der Bohrnadel (2') am Umfang des Objektes (1) und einer Stelle (4') der vorbestimmten Einführtiefe umfasst, und wobei die von der Bohrnadel (2') induzierten Impulse Impulswellen (5) erzeugen, deren sich zum Umfang (1) des Objekts ausbreitende Anteile von den Sensoren (3) detektiert und der Impulslaufzeitmessung zugeführt werden.

9. Verfahren nach Anspruch 8, umfassend
c) Korrelieren der pro Streckenabschnitt (s₁,s₂,s₃) gemessenen Widerstandswerte der Bohrwiderstandsmessung mit den Impulslaufzeiten.

10. Verfahren nach zumindest einem der vorstehenden Ansprüche, wobei das Objekt (1) aus einer Gruppe ist, die einen Baum, einen Holzmast, eine Säule, insbesondere eine Säule aus natürlichem Stein oder aus Stein enthaltendem Werkstoff, aus Beton oder einer Werkstoffkombination aus Stein und Beton, umfasst.

## Claims

1. A device for applying a method for examination of the condition of column-shaped or cylindrical objects (1), the device having
- a drilling apparatus (2) comprising a drill needle (2'),
- signal detection means (14) for drilling resistance measuring and pulse time delay measuring signals which can be generated or recorded by a drill needle (2') that can be inserted into an object to be examined, wherein the signal detection means (14) for the pulse time delay measuring signals have a plurality of pressure sensors (3) configured to be arranged on the object (1),
**characterised in that**
the drilling apparatus (2) has
- a location determining device,
- a compass, and
- an indicating device for an insertion angle (α) for a drilling resistance and pulse time delay measurement that is intended to be taken,
and comprises a computing unit (30) that can be handled separately from the drilling apparatus (2), wherein
the location determining device, the compass, the indicating device for the insertion angle (α) and/or the means (14) are operatively coupled to control electronics of the drilling apparatus (2), and
wherein the computing unit (30) and the signal detection means (14) each have a communication interface for establishing a communication connection for transmitting the signals generated or recorded by the drill needle (2') from the signal detection means (14) to the computing unit (30), wherein
the computing unit (30) has
- at least one interface for entering identification data of the object (1) to be examined, said data comprising at least one location of the object (1) that can be determined by means of the location determining device,
- a processor and storage unit that comprises a data processing programme and is configured to process the identification data of the object (1) to be examined and to predetermine for a user an examination mode for the object (1) to be examined and to evaluate the signals generated or recorded by the drill needle (2'), wherein the examination mode comprises predetermining a type of how to take the measurements, and
- an output unit for outputting the predetermined examination mode for the object (1) to be examined and for outputting evaluation results to the user.

2. The device in accordance with Claim 1,
**characterised in that**
the communication interface is a wireless interface to establish the data transmission connection via a local radio network.

3. The device in accordance with Claim 1 or 2,
**characterised in that**
the location determining device has a GPS receiver.

4. The device in accordance with any one of Claims 1 to 3, **characterised in that**
the device comprises a camera and
the computing unit (30) is a notebook, a smartphone, a tablet PC, a netbook, an iPad.

5. A method for examination of the condition of column-shaped or cylindrical objects (1) using the device in accordance with any one of Claims 1 to 4,
**comprising the steps of**
A) determining identification data, at least a location of the object (1), using the location determining unit, and recording the data by the computing unit (30),
B) outputting to a user an examination mode for the object (1) to be examined using the output unit and thereby predetermining a type of measuring the drilling resistance and the pulse time delay, a measuring height above ground (35) and an insertion angle (α), and a minimum number of the measurements to be taken on the object (1) to be examined, and
C) carrying out the examination according to the predetermined examination mode and supplying the data processing programme of the computing unit (30) with the recorded measurement data which at least are drilling resistance and pulse time delay measurement data, and evaluating the same, and
D) outputting the evaluation result to the user.

6. The method in accordance with Claim 5,
**comprising the steps of**
E) depending on the evaluation result from the computing unit (30), selecting whether the examination of the object (1) is to be continued and, if yes,
F) predetermining the examination mode and carrying out steps C) and D).

7. The method in accordance with at least any one of Claims 5 or 6, wherein the recorded measurement data comprise image recording data.

8. The method in accordance with any one of Claims 5 to 7,
wherein taking the drilling resistance and pulse time delay measurement comprises:
a) arranging the sensors (3) for recording pressure signals in at least one plane (E1, E2, E3) along a perimeter of the column-shaped or cylindrical object (1),
b) discontinuously inserting the drill needle (2') of the drilling apparatus (2) while taking the drilling resistance measurement down to a predetermined depth of penetration into the column-shaped or cylindrical section of the object (1) radially towards a longitudinal axis (A-A) of said section, wherein the discontinuous inserting comprises a pulse inducing stopping, followed by restarting the drill needle (2') after one or more sections (s₁, s₂, s₃) of a distance (s) between a point of insertion of the drill needle (2') along the perimeter of the object (1) and a point (4') of the predetermined insertion depth has been covered, and wherein the pulses induced by the drill needle (2') generate pulse waves (5) the portions of which that are spreading to the perimeter (1) of the object are detected by the sensors (3) and are supplied to the pulse time delay measurement.

9. The method in accordance with Claim 8, comprising:
c) correlating the resistance values of the drilling resistance measurement measured per section (s₁, s₂, s₃) with the pulse time delays.

10. The method in accordance with at least any one of the preceding claims, wherein the object (1) is from a group comprising a tree, a wooden pole, a column, more particularly a column consisting of natural stone or of a material containing stone, concrete or a material combination of stone and concrete.

## Revendications

1. Dispositif pour la réalisation d'un procédé d'examen de condition d'objets en forme de colonne ou cylindriques (1) qui présente
- une perceuse (2) avec une aiguille de perçage (2'),
- des moyens de détection de signal (14) pour des signaux de mesure de résistance de perçage et de mesure de temps de parcours d'impulsion qui peuvent être générés ou enregistrés par l'aiguille de perçage (2') pouvant être introduite dans un objet à examiner (1),
dans lequel les moyens de détection de signal (14) pour les signaux de mesure de temps de parcours d'impulsion présentent plusieurs capteurs de pression (3) qui sont réalisés pour l'agencement sur l'objet (1), **caractérisé en ce que**
la perceuse (2) présente
- un dispositif de détermination de position,
- une boussole et
- un dispositif d'affichage pour un angle d'introduction (a) d'une mesure de résistance de perçage et de temps de parcours d'impulsion à réaliser, et comprend un module de calcul (30) pouvant être manipulé séparément de la perceuse (2), dans lequel
le dispositif de détermination de position, la boussole, le dispositif d'affichage pour l'angle d'introduction (a) et/ou les moyens (14) sont couplés de manière opérationnelle à une électronique de commande de la perceuse (2), et
dans lequel le module de calcul (30) et les moyens de détection de signal (14) présentent chacun une interface de communication pour l'établissement d'une liaison de communication pour la transmission des signaux générés ou enregistrés par l'aiguille de perçage (2') des moyens de détection de signal (14) au module de calcul (30), dans lequel le module de calcul (30) présente
- au moins une interface pour la saisie de données de caractérisation de l'objet à examiner (1) qui comprennent au moins une position de l'objet (1) pouvant être déterminée par le dispositif de détermination de position,
- un module de processeur et de mémoire avec un programme de traitement de données configuré pour le traitement des données de caractérisation de l'objet à examiner (1) et pour la prédéfinition d'un mode d'examen pour l'objet à examiner (1) à un utilisateur et pour l'analyse des signaux générés ou enregistrés par l'aiguille de perçage (2'), dans lequel le mode d'examen comprend la prédéfinition d'une manière d'effectuer les mesures, et
- un module d'émission pour l'émission du mode d'examen prédéfini pour l'objet à examiner (1) et pour l'émission de résultats d'analyse à l'utilisateur.

2. Dispositif selon la revendication 1,
**caractérisé en ce que**
l'interface de communication est une interface radio pour l'établissement de la liaison de transmission de données par le biais d'un réseau radio local.

3. Dispositif selon la revendication 1 ou 2,
**caractérisé en ce que**
le dispositif de détermination de position présente un récepteur GPS.

4. Dispositif selon au moins une des revendications 1 à 3, **caractérisé en ce que**
le dispositif comprend une caméra, et
le module de calcul (30) est un ordinateur portable, un smartphone, des tablettes, des mini-ordinateurs portables, un i-Pad.

5. Procédé d'examen de condition d'objets en forme de colonne ou cylindriques (1) en utilisant le dispositif selon une des revendications 1 à 4,
**comprenant les étapes de**
A) détermination de données de caractérisation, au moins d'une position de l'objet (1), avec le module de détermination de position et détection des données par le module de calcul (30),
B) émission d'un mode d'examen pour l'objet à examiner (1) à un utilisateur avec le module d'émission, prédéfinition ce faisant d'au moins une méthode de mesure de résistance de perçage et de temps de parcours d'impulsion, d'une hauteur de mesure au-dessus du niveau du sol (35) et d'un angle d'introduction (α), et d'un nombre minimal des mesures à réaliser sur l'objet à examiner (1), et
C) réalisation de l'examen en fonction du mode d'examen prédéfini et acheminement des données de mesure enregistrées, qui sont au moins des données de mesure de résistance de perçage et de temps de parcours d'impulsion, au programme de traitement de données du module de calcul (30) et analyse de celles-ci, et
D) émission du résultat d'analyse à l'utilisateur.

6. Procédé selon la revendication 5,
**comprenant les étapes**
E) sélectionner par le module de calcul (30) en fonction du résultat d'analyse si l'examen sur l'objet (1) est à poursuivre, si c'est le cas
F) prédéfinition du mode d'examen et réalisation des étapes C) et D).

7. Procédé selon au moins une des revendications 5 ou 6,
dans lequel les données de mesure enregistrées comprennent des données d'enregistrement d'image.

8. Procédé selon au moins une des revendications 5 à 7,
dans lequel la réalisation de la mesure de résistance de perçage et de temps de parcours d'impulsion comprend :
a) agencement des capteurs (3) pour l'enregistrement de signal de pression dans au moins un plan (E1, E2, E3) à une périphérie de l'objet en forme de colonne ou cylindrique (1),
b) l'introduction discontinue de l'aiguille de perçage (2') de la perceuse (2) en réalisant la mesure de résistance de perçage jusqu'à une profondeur de pénétration prédéterminée radialement en direction d'un axe longitudinal (A-A) de la section dans la section en forme de colonne ou cylindrique de l'objet (1), dans lequel l'introduction discontinue comprend un arrêt induisant une impulsion, suivi d'un redémarrage de l'aiguille de perçage (2') après avoir parcouru une ou plusieurs sections de trajet (si, s₂, s₃) sur un trajet (s) entre un point d'introduction (4) de l'aiguille de perçage (2') à la périphérie de l'objet (1) et un point (4') de la profondeur d'introduction prédéterminée, et dans lequel les impulsions induites par l'aiguille de perçage (2') génèrent des ondes d'impulsion (5) dont des portions se propageant vers la périphérie (1) de l'objet sont détectées par les capteurs (3) et acheminées à la mesure de temps de parcours d'impulsion.

9. Procédé selon la revendication 8, comprenant
c) mise en corrélation des valeurs de résistance mesurées par section de trajet (si, s₂, s₃) de la mesure de résistance de perçage avec les temps de parcours d'impulsion.

10. Procédé selon au moins une des revendications précédentes, dans lequel l'objet (1) provient d'un groupe qui comprend un arbre, un poteau en bois, une colonne, notamment une colonne en pierre naturelle ou en matériau contenant de la pierre, en béton ou une combinaison de matériaux de pierre et béton.
